# EUROPEAN PATENT APPLICATION

(11) **EP 2 075 322 A1**
(43) Date of publication of application: **01.07.2009**
(21) Application number: 07024602.0
(22) Date of filing: 19.12.2007
(51) Int. Cl.: C11C 3/10, C07C 67/08, C08F 220/20

(54) **Triglyceride macromonomers**

(71) Applicant: Akzo Nobel Coatings International B.V., 6824 BM Arnhem (NL)
(72) Inventor: Williams, Neal, Warfield Berkshire RG42 3JN (GB); Lampard, Chris, Slough, Berkshire SL1 6EB (GB); Carter, Jeff T, Marton Middlesborough Cleveland TS8 9QR (GB); Quinn, Steve, Darlington DL3 9AU (GB)
(74) Representative: Schalkwijk, Pieter Cornelis

(57) **Abstract**

An ethylenically unsaturated macromonomer being the reaction product of
i) an adduct formed from the reaction of an unsaturated non-mineral oil reacted with an enophile having an acid, ester or anhydride moiety and
ii) an ethylenically unsaturated monomer having a moiety reactive with the acid, ester or anhydride moiety of the enophile and
iii) a chain extender material having at least two moieties reactive with the acid, ester or anhydride moiety of the enophile.

## Description

This invention relates to ethylenically unsaturated macromonomers derived from unsaturated non-mineral oils-especially vegetable oils; polymers-particularly in the form of aqueous dispersions derived from them and finally adhesives, coatings, especially architectural coatings, comprising the polymers.

By architectural coatings is meant paints, varnishes and woodstains, especially for use on the interior and exterior of structures and buildings such as houses, and also coatings for use in the garden such as fence paints and also metal used on railings.

There is increasing awareness of the need to use renewable resources in industry.

For many years, the main feedstock for industry, especially the chemical industry, has been fossil feedstock, either in the form of petroleum or coal. Whilst economists, mining and oil exploration experts may argue about the lifetime of known and yet to be discovered fossil feedstock reserves, it is indisputable that, in time, those reserves will inevitably be exhausted and even prior to that, become too expensive to be of any use as a feedstock in all but the most specialised applications.

Furthermore, converting this fossil feedstock into useful material for industry requires energy, releasing carbon dioxide and contributing to global warming. In addition, at the end of the useful life of the material, still more carbon dioxide is released, further adding to global warming.

Coatings typically comprise a mixture of particulate inorganic material and organic material. The inorganic material is usually a mixture of pigments and fillers and/or extenders. The pigments give colour to the paint and the fillers and extenders provide other properties, such as hardness, to the paint film. The organic material largely comprises a binder, the role of which, as the name suggests, is to bind any particulate matter together. It also provides adhesion to the surface to which the coating is applied.

The binders are usually high molecular weight polymers derived from monomers obtained by refining and further processing of fossil feedstock. The polymers are often produced in the form of aqueous dispersions of polymer microparticles referred to as latex. For simplicity it should be understood that the term polymer is used here to include homopolymers, copolymers, terpolymers and so on.

The manufacture and use of such polymer binders consumes some of the worlds non-renewable resources and in the process produces carbon dioxide.

Thus there is a need for polymer binders that are based, at least in part, on renewable feedstock such as that obtained from plant and/or animal material.

Oils are such a natural and renewable feedstock obtainable from various plants and animals, including fish. In the case of plants, it is generally the fruit that yields the oil. The term oil as used here, excludes mineral oils obtained form fossil feedstock such as petroleum and coal.

These oils are largely composed of a mixture of triglycerides (ie tri-esters of glycerol) characterised by the fatty acids portion. For example, the fatty acids component of the triglycerides making up palm oil, linseed oil and soya oil are shown below and have the following approximate composition by weight %:

| | Palm oil | Linseed oil | Soya oil |
|---|---|---|---|
| Palmitic acid (C₁₆, 0) | 44 | 6 | 10 |
| Stearic acid (C₁₈, 0) | 4.5 | 2.5 | 4 |
| Arachidic (C₂₀, 0) | 0 | 0.5 | 0 |
| Oleic acid (C₁₈, 1) | 40 | 19 | 23 |
| Linoleic (C₁₈, 2) | 10 | 24.1 | 51 |
| Linolenic (C₁₈, 3) | 0 | 47.4 | 7 |
| Other | 1.5 | 0.5 | 5 |
| | | | |
| Saturated | 48.5 | 9 | 14 |
| Unsaturated (total) | 50.0 | 90.5 | 81 |
| Unsaturated (>1) | 10.0 | 71.5 | 58 |
| Iodine value | 44-54 | 155-205 | 120-141 |

The subscript refers to the carbon chain length of the fatty acid and 0, 1 or 2 indicates the number of ethylenically unsaturated bonds in the fatty acid.

Such unsaturated oils can be classified as drying, non-drying or semi-drying oils. What is meant by such terminology is the extent to which they autoxidise at normal temperatures to form a hard, dry film. Autoxidation is the process by which unsaturated oils absorb oxygen from the atmosphere to form in-situ hydroperoxides which then decompose to produce free radicals causing the oils to dry. The more unsaturated bonds the oil has, the more rapidly and completely it will dry. Similarly, oils with at least two unsaturated bonds per triglyceride, especially when conjugated, autoxidise even more readily.

The extent of the unsaturation is measured by the iodine value. It is generally regarded that non-drying oils have iodine values of less than 100, semi-drying oils from 100 to 140 and drying oils over 140 g of iodine per100g of oil. A more extensive list of iodine values can be found on pages 34 and 35 of The Chemistry of Organic Film Formers by D.H.Solomon, published by John Wiley and Sons in 1967, which are hereby incorporated by reference.

The oils can be characterised according to the number of double bonds per triglyceride type. We have found that a typical sample of palm oil, for example, has the following distribution, expressed on a weight % basis.

| | |
|---|---|
| 5 double bonds per triglyceride | <1 |
| 4 double bonds per triglyceride | ca 10 |
| 3 double bonds per triglyceride | ca 5 to 10 |
| 2 double bonds per triglyceride | ca 35 |
| 1 double bonds per triglyceride | ca 35 to 40 |
| 0 double bonds per triglyceride | ca 10 |

Thus whilst approximately 48.5% of the fatty acids in the oil are palmitic or stearic and thus saturated, nevertheless only about 10% of the triglycerides will be composed of fatty acids where all three are saturated, and therefore free of double bonds. This saturated portion of the palm oil is unreactive to enophile and dienophiles.

US 20050203246 discloses monomer formed by reacting unsaturated vegetable oils (specifically sunflower oil, linseed oil or soya bean oil) with an enophile or dienophile having an acid, ester or anhydride functionality, to form a derivative followed by reacting the derivative with a suitable hydroxyl, amine, thiol, oxirane or other functional vinyl monomer to form an ethylenically unsaturated triglyceride monomer. The weight average molecular weight of such monomers is approximately 1000 Daltons. It also discloses polymer latices containing up to 6% by weight of the monomer calculated on the polymer solids

However, we have found that latices derived from monomer mixtures comprising such triglyceride monomer, and paints based on them, are unusable as they exude oily material. This is especially problematic when the monomer comprises more than 10% by weight of the polymer. This significantly limits the amount of the monomer that can be used in a coating composition. Consequently, the benefit of using such a renewable resource cannot be exploited to the full extent.

In addition, architectural coatings using binders comprising such monomers, especially those used on interior surfaces, tend to have a yellow hue. This makes it difficult to formulate bright whites and the pastel colours.

These problems of the known prior art severely limits the utility of these known triglyceride monomers.

We have now developed a new macromonomer based on unsaturated non-mineral oils obtained from plant or animal material which can be used in much higher amounts in polymer binders and with much reduced or no exudation of oily material.

According to the present invention there is provided an ethylencially unsaturated macromonomer being the reaction product of
i) the adduct formed from the reaction of an unsaturated non-mineral oil reacted with an enophile having an acid, ester or anhydride moiety, preferably maleic anhydride and
ii) an ethylenically unsaturated monomer having a moiety reactive with the acid, ester or anhydride moiety of the enophile and
iii) a chain extender material having at least two moieties reactive with the acid, ester or anhydride moiety of the enophile.

Preferably the weight average molecular weight, Mw, of the macromonomer is 1000 to 50000 Daltons, more preferably from 5000 to 40000, still more preferably from 10000 to 30000 Daltons, yet more preferably from 17000 to 25000 Daltons and most preferably form 19000 to 23000 Daltons.

For simplicity, the term enophile is used to include dienophile.

Enophiles have electron withdrawing moieties, such as ester, acid, cyanide and anhydride. Preferably the enophile is an electrophillic alkene or alkyne. Even more preferably is selected from the group consisting of maleic anhydride, fumaric acid, itaconic anhydride, acrylic acid and maleate esters and most preferably it is selected from the group consisting of maleic anhydride and fumaric acid.

By non-mineral oil is meant oil, comprising triglycerides, that has been obtained directly from plant or animal matter, including fish, rather than from a fossil feedstock.

Preferably, at least 80% by weight of the total triglycerides making up the oil contain one or more double bonds, more preferably 85 to 100%, still more preferably 90 to 100%, yet more preferably 95 to 100% and most preferably 100%. The amount of fully saturated triglycerides is preferably kept as low as possible as such triglycerides are unreactive to the enophile. The oily exudate seen on the surface of dried latex and paint films is thought to be this unreacted saturated material.

Being natural products, the composition of the oils varies significantly from year to year, the geographic location of the source and the degree of any further processing that may be carried out on the oil. Blending oils from different sources and even of different types not only produce a more consistent feedstock, but also enables oils having a triglyceride composition not found in nature to be produced.

However, even such blends are not ideal, because they consist of a complex mixture of triglycerides having saturated and mono and poly-unsaturated fatty acid portions. This inevitably results in a distribution of species being formed when the oils are subsequently reacted with polyfunctional material. There is also a risk of gelation. The ideal feedstock is one where the oil consists of a triglyceride having one monounsaturated and two saturated fatty acid moieties but this is not available in nature.

Transesterification of oils, whereby the fatty acid portions between and within triglycerides are rearranged, can produce oils which, whilst not composed of the ideal triglyceride structure described above, nevertheless have more of the triglycerides with fewer unsaturated bonds, than the naturally occurring oil. This significantly reduces the risk of gelation during manufacture of the macromonomer.

Transesterification can be carried out with a single oil, resulting in randomisation of the fatty acid composition of the triglycerides. Or it may be performed with a mixture, comprising two or more oils. Careful choice of the oil mixture to be intersterified allows the triglyceride composition to be controlled.

An additional benefit of transesterification is that by blending oils and transesterifying the resulting mixture, a more consistent feedstock can be produced for further reaction according to the invention.

Accordingly, there is provided a method of making an ethylenically unsaturated macromonomer comprising the steps of
i) providing a chosen mixture of unsaturated non-mineral oils having a first blend of triglycerides
ii) treating the mixture of oils with an transesterification catalyst to produce a second blend of triglycerides different to the first
iii) reacting the second blend of triglycerides with an enophile having an acid, ester or anhydride moiety to form an adduct
iv) reacting the adduct with an ethylencially unsaturated monomer having a moiety reactive with the acid, ester or anhydride moiety of the enophile to form a low molecular weight monomer
v) reacting the monomer of step iv) with a chain extender having at least two moieties reactive with the acid, ester or anhydride moiety of the enophile to form the ethylenically unsaturated macromonomer.

Preferably, the macromonomer is diluted with a suitable solvent, preferably one that is also a polymerisable monomer in a final step. This ensures that the macromonomer does not solidify on cooling down from the reaction temperature and thereby become difficult to handle. 2-ethyl hexyl acrylate is a suitable such solvent/monomer.

Transesterification may be performed in the presence of a suitable catalyst at about 60 to 80°C for approximately half an hour. Suitable catalysts include sodium methoxide, sodium ethoxide, sodium isopropxide, sodium hydroxide, potassium hydroxide, sulphated zirconia, tungstated zirconia, hydrochloric acid, sulphuric acid, boron trifluoride, sodium ethylate, stannium hydroxide, potassium glycerolate, sodium methylate and lipases. Preferably, from 0.05 to 1.5% by weight of the catalyst is used calculated on the total amount of oil to be transesterified, more preferably from 0.05 to 1.0%, still more preferably from 0.1 to 0.5% and most preferably from 0.1 to 0.3%. Preferably, sodium methoxide is used as this is easily removed following the reaction. When sodium methoxide is used as the catalyst it is preferred to dry the oil and/or oils in order to prevent its hydrolysis. Preferably, the oil used to manufacture the macromonomer is derived from such transesterified oils.

Plant oils are preferred as their production consumes carbon dioxide rather than producing it.

Preferably the iodine value of the oil is less than 140 as the initial colour of the polymer film derived from it is less yellow. More preferably the iodine value is between 30 and 140, even more preferably from 30 to 100, still more preferably from 35 to 70 and most preferably from 40 to 60.

The proportion of the fatty acid component (of the triglycerides in the oil) having more than one double bond, is preferably less than 15%, more preferably from 0.5 to 15%, even more preferably from 1 to 12%, still more preferably from 1 to 10% and yet more preferably from 2 to 10% and most preferably from 5 to 10% by weight. It is such polyunsaturated fatty acids, especially having conjugated double bonds, which produce yellowing in dried paint films.

Suitable oils are those having iodine value of between 30 and 140. They may be used alone or blended with others include Palm oil, Soya bean oil, Cotton seed oil, Kapok oil, Mustard oil, Olive oil, Peanut oil, Rapeseed oil, Sesame oil and hydroxyl functional Castor oil. Preferably the oils for use in the invention are selected from the group consisting of Palm oil, Soya bean oil, Cotton seed oil, Kapok oil, Mustard oil, Olive oil, Peanut oil, Rapeseed oil, Sesame oil and hydroxyl functional Castor oil.

Palm oil is a useful oil as the macromonomer derived from it has a good balance of reactivity and resistance to yellowing, especially when formulated into a polymer latex for use as a binder in a coating composition. Whilst Palm oil, like the other oils, is a complex mixture of species, nevertheless the molecular weight is taken to be 848 Daltons for the purposes of stochiometric calculations.

Where it intended to produce a mixture of triglycerides by transesterification of a mixture of oils, oils of greater unsaturation, having iodine value greater than 140, may also be used in the transesterification reaction itself. However, such a mixture of oils should consist of less than 80% by weight, more preferably less than 70% and even more preferably less than 60% of the total oil mixture calculated on a weight basis. Such oils include Corn oil, Safflower oil, Sunflower oil and Tall oil.

Preferably, the moiety on the ethylenically unsaturated monomer, reactive with the acid, ester or anhydride moiety of the enophile is a hydroxyl, amino or epoxide. Even more preferably, there is only one such moiety on the monomer.

Preferably the ethylenically unsaturated monomer reactive with the acid, ester or anhydride moiety of the enophile is selected from the group comprising hydroxyethyl (meth)acrylate, hydroxyl propyl (meth)acrylate, hydroxyl iso propyl methacrylate, hydroxyl butyl methacrylate, allyl alcohol, glycerol methacrylate, glycidyl (meth)acrylate, allyl amine, tert-butyl aminoethyl methacrylate. More preferred are hydroxy ethyl acrylate and hydroxyl ethyl methacrylate and most preferred is hydroxyl ethyl methacrylate.

Preferably the chain extender material comprises hydroxyl, amine, oxirane or isocyanate moieties. More preferably, it comprises at least two moieties capable of reacting with the acid, ester or anhydride moiety of the enophile as this reduces the possibility of gelation through crosslinking. Nevertheless, it is possible that a trifunctional material is acceptable if one of the moieties is much slower to react than the other two. For example, this is thought to be the case when glycerol is used, where the secondary hydroxyl group is less reactive than the two primary hydroxyl groups. Preferably, the chain extender material is glycerol as it is readily obtained from sustainable plant material.

The mole ratio of oil:enophile is preferably less than or equal to 1, more preferably from 0.50 to 1.00, even more preferably from 0.55 to 0.75 and most preferably from 0.60 to 0.70. The resulting excess of enophile helps to reduce the amount of unreacted oil thereby limiting the tendency to produce exudate.

The mole ratio of oil:chain extender is preferably greater than 1.8, more preferably from 1.8 to 10.0, even more preferably from 2.0 to 7.5, still more preferably from 2.5 to 5 and most preferably from 3.0 to 3.5.

The mole ratio of oil:enophile:unsaturated monomer:chain extender is preferably 1.00:1.50:0.75:0.30.

The reaction of the oil with the enophile is thought to produce an adduct of the triglycerides comprising the oil, and the enophile. The adduct having an acid, ester or anhydride moiety reacts with a suitable functional moiety on the ethylenically unsaturated monomer to form a polymerisable triglyceride monomer. Further reaction with a chain extender material, increases the molecular weight of the triglyceride monomer.

Preferably the molecular weight of the resulting macromonomer is at least twice that of the triglyceride monomer.

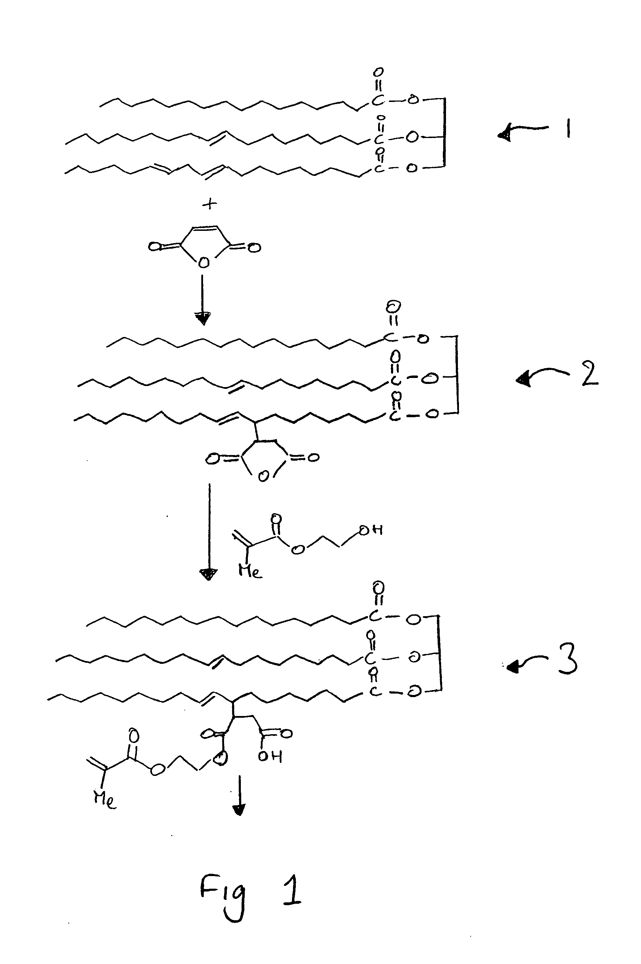

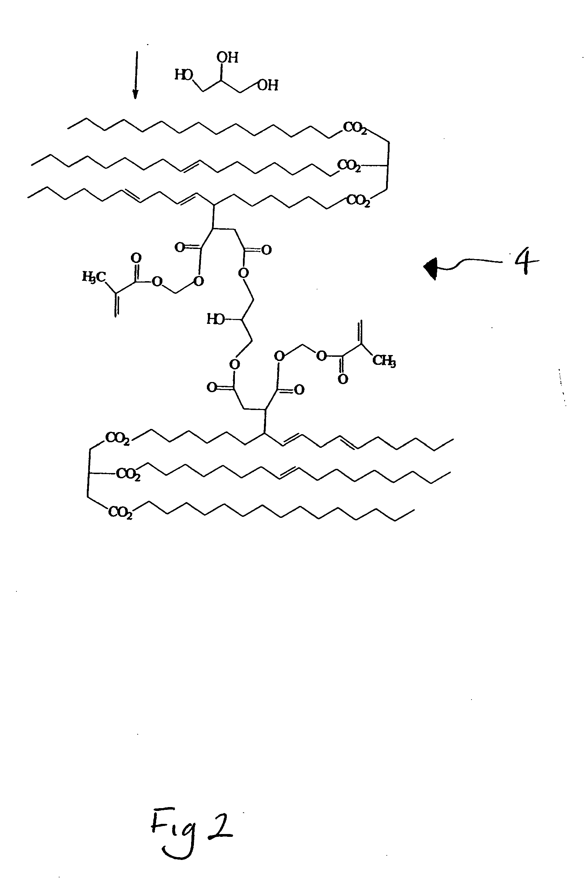

Figure 1 shows an idealised diagrammatic reaction scheme of an unsaturated oil comprising a triglyceride, (1), reacted with maleic anhydride as the enophile to form the adduct (2) and then reacted with hydroxyl ethyl methacrylate to form a triglyceride monomer (3). Figure 2 shows the triglyceride monomer (3) reacted further with glycerol as the chain extender material to form the macromonomer (4) of the invention.

Of course, because of the numerous multi-functional species formed during the reaction, the measured molecular weight of the macromonomer is much higher than as depicted in the drawings and rather is as hereinbefore described.

The oil is represented by an idealised triglyceride structure (1) comprising palmitic acid, oleic acid and linoleic acid.

In a further aspect of the invention there is provided a process of making the macromonomer comprising the steps of
i) reacting an unsaturated non-mineral oil with an enophile having an acid, ester or anhydride moiety to form an adduct
ii) reacting the adduct with an ethylencially unsaturated monomer having a moiety reactive with the acid, ester or anhydride moiety of the enophile to form a low molecular weight polymerisable monomer
iii) reacting the monomer of step ii) with a chain extender having at least two moieties reactive with the acid, ester or anhydride moiety of the enophile to form the ethylenically unsaturated macromonomer.

Preferably the molecular weight of the macromonomer formed in step iii) is as previously defined.

The reaction of step i) is preferably carried out above 180°C, more preferably from 200 to 300°C and most preferably from 200 to 250°C.

Where the fatty acid component of the triglycerides comprising the oil contains less than 30% of fatty acids having conjugated double bonds, it is preferred to heat the oil in the presence of iodine at a temperature of from 80 to 120°C, more preferably from 90 to 110°C and most preferably 100°C. The presence of the iodine serves to convert the unconjugated double bonds, for example of linoleic acid, to conjugated double bonds which are thus able to react with the enophile and/or dienophile, for example maleic anhydride, via the preferred Diels Alder reaction. Preferably the ratio of iodine:oil is from 0.002:1 to 0.010:1 calculated on a molar basis. We have found that macromonomer produced including this step produces less yellowing in a polymer binder comprising the macromonomer than binder derived from macromonomer produced without the iodine step. This is even more evident when such binders are used to make pastel or white paint.

Since the reaction of the enophile with the unsaturated bonds requires the temperature to be at least 180°C and preferably from 200 to 250°C, the enophile may be present during the oil and iodine heating stage. Preferably, the temperature of the reactants is kept below 120°C.

Depending on the choice of unsaturated oil, the resultant neat macromonomer can vary in appearance at room temperature from a reasonably fluid liquid to a greasy or even waxy consistency. The oils of low iodine value, say less than 100, tend to be soft solid since they are less saturated than the higher iodine value oils.

In a further aspect of the invention, there is provided an addition polymer comprising the macromonomer as hereinbefore described. Preferably the polymer is polymerised with other ethylenically unsaturated monomers. Examples of suitable ethylenically unsaturated monomers include (meth)acrylic acid esters, amides, and nitriles, vinyl monomers and vinyl esters.

Using the nomenclature of (meth)acrylate to represent both acrylate and methacrylate, examples of suitable other acrylic acid esters and methacrylic acid esters are alkyl esters, preferably methyl (meth)acrylate, ethyl (meth)acrylate, propyl (meth)acrylate, butyl (meth)acrylate, 2-ethyl hexyl (meth)acrylate and alkoxy poly(oxyethylene) (meth)acrylate.

Small amounts of methacrylic acid and/or acrylic acid may also be used. Hydroxy functional monomers such as hydroxy ethyl (meth)acrylate and hydroxy isopropyl (meth)acrylate may be included also. Examples of suitable vinyl monomers include styrene and alpha methyl styrene, vinyl propionate, vinyl butyrate, vinyl acetate and vinyl versatate. Preferably the addition polymer is derived from the esters of acrylic acid, methacrylic acid and optionally styrene and/or its derivatives. More preferably, the other monomers are styrene and 2-ethyl hexyl acrylate.

The glass transition temperature, or Tg, of the polymer comprising the macromonomer of the invention may be varied by copolymerising monomers of appropriate Tg. In this way copolymers which are hard, soft or of intermediate Tg can be made., which can produce a wide range of physical film properties such as tack (or stickiness), hardness and extensibility. Due to the long alkyl chains of the macromonomers, the Tg's will be low, comparable to butyl acrylate, lauryl acrylate and stearyl acrylate depending on the starting oil.

Preferably the Tg of the polymer is from -70 to 185°C, more preferably from -20 to 120°C. More preferably the polymer is suitable for use as a binder in coatings especially in architectural coating compositions. For such binder polymer dispersions the preferred range is from -20 to 120°C, yet more preferably from -15 to 60°C, even more preferably from -10 to 25°C and most preferably from -10 to 10°C as this produces a more durable paint which is better resistant to knocks and scuffs.

More preferably, the polymer is in the form of an aqueous composition and still more preferably is an aqueous dispersion of polymer microparticles. By aqueous is meant that at least 50% by weight of the carrier liquid is water, more preferably more than 75% and most preferably the carrier liquid is water.

Preferably the polymer microparticles have a mean diameter of from 0.05 to 2 microns, more preferably from 0.05 to 1.0 microns, still more preferably from 0.05 to 1.0 microns and most preferably from 0.05 to 0.3 microns.

Optionally, the microparticles are of the core-shell type having a core polymer composition different from the shell polymer composition. Preferably, the core:shell ratio calculated on a weight basis is from 1:8 to 2:1, more preferably from 1:2.

The ethylenically unsaturated monomers are caused to copolymerise by heating the monomer, in a carrier liquid-preferably water, containing polymerisation initiators, preferably to a temperature of from 30°C to 150°C, preferably from 40°C to 80°C. More preferably the polymerisation process used is an aqueous emulsion polymerisation process. In such a case the maximum polymerisation temperature should not exceed 98°C. Even more preferably, where redox initiator combinations are used, the preferred polymerisation temperature is from 20°C to 80°C and most preferably from 30°C to 70°C.

Suitable emulsion polymerisation initiators include oxidants, for example, peroxides such as tertiary butyl hydroperoxide, hydrogen peroxide and cumene hydroperoxide; persulphates such as potassium persulphate and ammonium persulphate; azo types such as 4,4' azobis (4-cyanopentanoic acid). Preferably from 0.002% by weight to 5% by weight of the initiator is used, calculated on the amount of ethylenically unsaturated monomers, more preferably from 0.05 to 2% and most preferably from 0.1 to 1%.

Reductants may be used in combination with the oxidant to form so called redox couples. This enables the polymerisation to be run at lower temperature than when relying on thermal decomposition alone. Suitable examples of such oxidants include sodium ascorbate, sodium metabisulphite and sodium formaldehyde sulphoxylate. Suitable examples of redox couples include tertiary butyl hydroperoxide with ascorbic acid or sodium ascorbate or sodium metabisulphite or sodium formaldehyde sulphoxylate; hydrogen peroxide with ascorbic acid, sodium ascorbate or sodium metabisulphite or sodium formaldehyde sulphoxylate; cumene hydroperoxide with ascorbic acid sodium ascorbate or sodium metabisulphite or sodium formaldehyde sulphoxylate. More preferred is the redox couple tertiary butyl hydroperoxide with sodium ascorbate.

Optionally, metal salts such as copper, chromium and iron salts can be added when redox pairs are used. Such metals, usually in the form of water soluble salts, for example iron(II) sulphate, are especially useful where the natural level of dissolved metals in the reaction mixture are low. This can occur when a glass-lined reactor is used or a metal chelating agent is present. The presence of the added metal salts ensures that the redox system works effectively. Preferably the level of added metal salt is kept to a minimum to avoid discolouration of the dispersion itself and any coatings derived from it. This is generally less of a problem for adhesives.

The preferred initiator system is the redox combination of tertiary butyl hydroperoxide and ascorbic acid, the latter optionally in the form of sodium ascorbate. Such redox combinations allow the polymerisation to be carried out or around ambient temperature such as from 30 to 55°C.

In a further aspect of the invention there is provided a process of producing an aqueous dispersion of polymer microparticles derived from a monomer mixture comprising the macromonomer and other ethylenically unsaturated monomers, as hereinbefore described.

Preferably the process comprises the steps of
i) making an emulsion of ethylenically unsaturated monomers comprising the macromonomer and other monomers, in aqueous medium, preferably water, containing surfactant
ii) charging from 5 to 25% by weight of the monomer emulsion to a polymerisation vessel and causing it to polymerise of to form polymer microparticles of mean particle diameter of from 0.05 to microns
iii) feeding the remaining monomer emulsion of step i) to the vessel in the presence of the microparticles of step ii) and causing it to polymerise and grow them to form the final microparticles of mean particle diameter of from 0.05 to 2.0 microns.

More preferably, the process comprises the steps of
i) making an aqueous emulsion comprising the macromonomer and no other monomer
ii) charging the emulsion to a polymerisation vessel and causing it to polymerise to form polymer microparticles of mean particle diameter of from 0.05 to 2 microns
iii) feeding a neat mixture of the other monomers to the vessel in the presence of the microparticles of step ii) and causing it to polymerise and grow them to form the final microparticles.
   This has the advantage that there is no need to make an aqueous emulsion of the macromonomer and the other ethylenically unsaturated monomers thereby saving time.

It is thought that this produces a core-shell structure to the particle.

In a yet further aspect of the invention there is provided a coating composition comprising the addition polymer as hereinbefore described. Preferably, the coating is aqueous. More preferably the coating is an architectural paint for use on the interior and exterior surfaces of structures such as found in homes, offices and gardens. Even more preferably, the paint is of non-Newtonian rheology, even more preferably, thixotropic rheology.

The coating composition may also contain ingredients selected from the group consisting pigments, fillers, waxes, extenders, rheological modifiers, dispersants, anti-foams, plasticisers, crosslinking agents, flow aids and biocides.

### Materials used in the examples

RD Palm Oil is available from Hampshire Commodities, Fleet, Hampshire, UK, GU51 3SR.

Sunflower oil is available from
Hydrogenated Soya wax flake available from Cargill
tert-butyl hydroperoxide (t-BHP) is available from Akzo-Nobel
Disponil A4066 is available from Henkel

### Tests

### Iodine Value

Molecular Wight was determined by Gel Permeation Chromatography (GPC) using a Waters 150CV fitted with a refractive index detector. The columns used were 2 x 30cm PLGel Mix D GPC columns at a temperature of 35°C. Polystyrene (ex. Polymer Laboratories) was used as the standard. The test material was dissolved in tetrahydrofuran at a concentration of 1mg cm⁻³. The flow rate was 1 cm³ min⁻¹.

Examples of the invention will now be described.

### Macromonomer Example (MM 1)

| | g | wt % |
|---|---|---|
| RD Palm oil | 1698.59 | 56.620 |
| Maleic anhydride | 294.30 | 9.820 |
| Iodine | 2.55 | 0.085 |
| Phenothiazine | 3.38 | 0.113 |
| Hydroxy ethyl methacrylate | 195.51 | 6.517 |
| Glycerol | 55.33 | 1.845 |
| 2-ethyl hexyl acrylate | 250.00 | 25.000 |

To a 5 litre round bottomed flask fitted with a water cooled condenser, means to provide a nitrogen blanket and an anchor stirrer operating at 75 rpm was added the RD Palm oil and maleic anhydride whilst stirring. The mixture was heated to 100°C and the iodine was added; the temperature was held for 30 minutes. The temperature was raised to 200°C and held there for 30 minutes and then increased to 220°C. After 5 hours and thirty minutes it was cooled to 200°C and the phenothiazine was added. After five minutes the hydroxy ethyl methacrylate was added over a period of 15 minutes and held at 200°C for an hour, following which the glycerol was added. After an hour at 200°C the batch was cooled to 80°C and the 2-ethyl hexyl acrylate was added and then filtered through lambswool.

The resulting macromonomer dissolved in 2-ethyl hexyl acrylate is a clear, low viscosity liquid that is easy to handle.

### Latex Example (Latex 1)

A latex was made using the following ingredients and procedure

| | g | wt% |
|---|---|---|
| **Initiator** | | |
| t-BHP/1 | 5.48 | 0.498 |

| **Monomer Pre-emulsion (MPE)** | | |
|---|---|---|
| MM 1 | 171.73 | 15.612 |
| Styrene | 260.35 | 23.668 |
| 2-ethyl hexyl acrylate | 83.12 | 7.556 |
| Ammonia (25% solution) | 8.02 | 0.729 |
| Demin water/l | 444.42 | 40.402 |
| Disponil A4066 | 43.24 | 3.931 |
| | | |

| **Reductant Solution** | | |
|---|---|---|
| Demin water/2 | 77.80 | 7.073 |
| Sodium ascorbate | 3.10 | 0.282 |
| | | |
| t-BHP/2 | 2.74 | 0.249 |

Prepare 523.22g of Monomer Pre-emulsion by mixing the indicated weights of macromonomer MM 1, styrene, 2-EHA and adding the ammonia under gentle stirring for about 10 minutes, adding the ammonia dropwise. Add this monomer mixture to a solution of 444.42g of Demin/1 and 43.24g Disponil A4066 under high shear mixing conditions using a Silverson homogeniser to form an emulsion of monomer in water until the mean droplet size is about 150 nm and there are no monomer droplets greater than 2000 nm. This will require about 20 minutes.

Prepare the reductant solution by dissolving the sodium ascorbate in water

To a 21 parallel sided vessel fitted with a propeller stirrer, condenser and a nitrogen blanket, add 350.00g of the Monomer Pre-emulsion and raise the temperature to 50°C and add t-BHP/1. After 5 minutes at this temperature add 20.23g of the reductant solution. Allow the batch to exotherm for 5 minutes and then feed the remainder of the Monomer Pre-emulsion (MPE) linearly over 60 minutes whilst concurrently feeding 26.97g of the Reductant solution, linearly. Maintain at 50°C for a further 15 minutes and then add 6.74g of the Reductant solution and hold for a further 15 minutes. Add t-BHP/2 and maintain temperature for a further 5 minutes after which time add the remaining Reductant solution, 26.97g linearly over 30 minutes. Maintain at 50°C for 15 minutes and then cool and filter through 80 mesh nylon.

There was very little build-up on the reactor walls or the stirrer and the latex was bit free.

The latex constants were
pH: 7.2 subsequently adjusted to 8.5
weight solids: 50.0%
Mean particle size was 145 nm measured on a Malvern Mastersizer

### Paint Example

### Paint 1

A semi-gloss paint was made using Latex 1 and the ingredients listed below.

### Mill base stage

### Load to a clean dispersion vessel

| | |
|---|---|
| Tap water | 6.673 |
| Benzyl alcohol | 1.200 |
| Tamol 731 | 1.850 |
| Dispelair CF 823 | 0.173 |

### Start disperser on low speed and begin adding pigmentation.

### Increase speed as millbase thickens to maintain a good vortex.

| | |
|---|---|
| China clay supreme | 6.130 |
| Tioxide TR92 | 18.240 |

### Run 20 minutes. Check dispersion is less than 20µ.

### Then add the other materials adjusting speed as required.

| | |
|---|---|
| Dispelair CF823 | 0.173 |
| Product V189 | 0.020 |
| Tap water | 3.000 |
| Aquaflow NHS 300 | 0.750 |
| Acrysol SCT-275 | 0.750 |
| SUB-TOTAL | 38.959 |

### Run 10 minutes

### Cover and allow mill base to cool before making into finished product.

### Paint stage

### Load mill base to a clean vessel

| | |
|---|---|
| **Mill base** | 38.959 |
| **Add with stirring** | |
| Latex 1 | 44.152 |
| Tap water | 10.000 |
| Pre blend | |
| Tap water | 2.850 |
| Acrysol TT935 | 0.150 |

### Run 10 minutes Adjust to viscosity with

| | |
|---|---|
| Tap water | 3.889 |
| TOTAL | 100.000 |

### Paint Evaluation

The following tests were carried out on Paint 1

### Viscosity

9-10 poise measured at 25°C using a Rotothinner operating at 562 rpm.

### Gloss

The paint was drawn down on a glass panel, allowed to dry and the gloss measured at 60 and 85°
Gloss 60°: 48%
Gloss 85°: 88%

### Hardness

Erichsen Hardness after 7 days at ambient temperature: 38 seconds.

Fingernail hardness: slightly softer than standard

White index: 77.45

### Water resistance

### Wet scrub resistance: 4.87 mg/cm² after 2000 cycles

### Water spot

2 ml of tap water was dropped onto a 25 micron dried coat of the paint and covered with a watch glass and left to stand for 2 hours and assessed the paint assessed. No damage or permanent loss of gloss noted.

### Comparative Paint A

Same paint recipe as Paint 1 but X935-33 latex used

### Comparative Example A

Same as example 1 but without glycerol

### Transesterified Oil 1

A mixture of 360g of Sunflower oil and 240g of hydrogenated Soya wax was heated to 80°C in a rotary evaporator flask under a vacuum of 9 mbar for 30 minutes.

The dried oil was transferred from the evaporator to a 3-necked, 1 litre round bottomed flask fitted with a water condenser, a stirrer and source of nitrogen. The mixture was heated to 70°C under a nitrogen blanket and once on temperature 1.2g of sodium methoxide was added and the reaction allowed to proceed for 30 minutes. After this time 6g of deionised water was added to the flask and stirred for 5 minutes, following which 2g of Kieselguhr filter aid was added. The resulting mixture was passed at 70°C through a fine glass filter funnel. The starting and final oil mixture had the following mixture of triglyceride.

Table 1 shows the fatty acid composition of the triglycerides making up the starting sunflower oil and the soya wax and the final transesterified mixture. The composition was determined using Matrix Assisted Laser Desorption/Ionisation - Time of Flight (MALDI-TOF).

**Table 1**

| **Triglyceride composition** | **Sunflower oil** | **Hydrogenated Soya Wax** | **Transesterified oil 1** |
|---|---|---|---|
| OOO | 5.7 | 0 | 12.9 |
| LOO | 14.9 | 0 | 18.3 |
| LLO | 27.3 | 0 | 7.2 |
| LLL | 25.4 | 0 | 5.9 |
| LLLe | 0.2 | 0 | 0 |
| LLeLe | 0 | 0 | 0.1 |
| OOP | 2.1 | 0 | 7.5 |
| LOP | 5.3 | 0 | 3.7 |
| LLP | 9.0 | 0 | 4.8 |
| LLeP | 0.4 | 0 | 0 |
| LeLeP | 0 | 0 | 0 |
| OOS | 1.1 | 0.1 | 14.6 |
| OSP | 0.2 | 0 | 2.7 |
| OSS | 0 | 0.3 | 5.1 |
| OPP | 0.2 | 0 | 0.5 |
| LPP | 0.9 | 0 | 1.1 |
| SSS | 0 | 59.9 | 2.8 |
| PPP | 0 | 0.5 | 0.2 |
| SSP | 0 | 30.5 | 2.0 |
| SPP | 0 | 4.8 | 0.7 |
| Mono and disubstituted glycerides | 7.3 | 3.9 | 9.9 |
| Saturated | 0 | | 0 |
| Unsaturated (total) | 92.7 | | 84.4 |
| Unsaturated (>1) | 83.4 | | 41.0 |

| | | | |
|---|---|---|---|
| O represents oleic acid L represents linoleic acid Le represents linolenic acid P represents palmitic acid S represents stearic acid | | | |

OOO indicates the triglyceride having three oleic acid groups; OSP represents the triglyceride having oleic acid, stearic acid and palmitic acid.

MALDI-TOF analysis of the Interesterified oil 1 shows that the triglycerides having more than one ethylenic unsaturation reduces from approximately 83% to 41% by weight.

### Macromonomer Example MM 2

The same method was used in the manufacture of this example as used in example MM 1.

In this example the Maleic Anhydride: Oil ratio has been fixed at 1.5 and the Oil:Glyceol has been fixed at 0.22.

| | g |
|---|---|
| Interesterified oil | 450.00 |
| Maleic anhydride | 78.59 |
| Iodine | 0.69 |
| Phenothiazine | 1.98 |
| Hydroxy ethyl methacrylate | 52.56 |
| Glycerol | 13.48 |

450gms of the transesterified oil together with 78.59gms of maleic anhydride & a few drops of xylene where charged to a 1000ml 3-necked round bottomed flask. Also fitted to the flask was a nitrogen inlet designed to give a slight positive pressure to the system in order to maintain a nitrogen blanket. A thermocouple, mechanical stirrer & condenser were also fitted to the flask. The reactor contents were heated under stirring at 150rpm to 100°C at which point 0.69g solid iodine was added. The reaction was stirred at 100°C for 10 minutes after which the temperature was increased to 200°C. Once at 200°C the reaction was maintained at this temperature for 30 minutes. After this time the reaction temperature was increased to 220°C and held there for 4 hours after which it was cooled back to 200°C. Once at 200°C, 1.98gms of phenothiazine was added. This was allowed to mix for 5 minutes after which 52.56gms of 2-hydroxyethyl methacrylate (HEMA) was added from a dropping funnel over a 30 minute period. After the HEMA addition had been completed the reaction was held at 200C for 60 minutes. After the 60 minute hold 13.48gms of glycerol (pre-warmed to 50°C) was added to the reaction in a single shot. The reaction was again held at 200°C for a period of 60 minutes. The reaction was then allowed to cool to 50°C at which point the resultant macromonomer was poured into 2x 500ml jars containing a small quantity of molecular sieve.

## Claims

1. An ethylenically unsaturated macromonomer being the reaction product of
i) an adduct formed from the reaction of an unsaturated non-mineral oil reacted with an enophile having an acid, ester or anhydride moiety and
ii) an ethylenically unsaturated monomer having a moiety reactive with the acid, ester or anhydride moiety of the enophile and
iii) a chain extender material having at least two moieties reactive with the acid, ester or anhydride moiety of the enophile.

2. An ethylenically unsaturated macromonomer according to claim 1 wherein the oil has an iodine value between 30 and 140.

3. An ethylenically unsaturated macromonomer according to claim 1 or claim 2 wherein the oil is obtained from plant matter.

4. An ethylenically unsaturated macromonomer according to any one of the previous claims wherein the oil is palm oil.

5. An ethylenically unsaturated macromonomer according to any one of the previous claims wherein the enophile is selected from the group consisting of maleic anhydride, itaconic anhydride, fumaric acid, acrylic acid and maleate esters.

6. An ethylenically unsaturated macromonomer according to any one of the previous claims wherein the monomer reactive with the acid, ester or anhydride of the enophile is selected from the group consisting of hydroxyl ethyl (meth)acrylate, hydroxyl propyl (meth)acrylate, hydroxyl iso propyl methacrylate, hydroxyl butyl methacrylate, allyl alcohol, glycerol methacrylate, glycidyl (meth)acrylate, allyl amine, tert-butyl aminoethyl methacrylate.

7. An ethylenically unsaturated macromonomer according to any one of the preceding claims wherein the chain extender is glycerol.

8. An ethylenically unsaturated macromonomer according to any one of the preceding claims wherein the molar ratio of oil:enophile:unsaturated monomer:chain extender material is 1:1.5:0.75:0.30.

9. An ethylenically unsaturated macromonomer according to any one of the preceding claims having a weight average molecular weight, Mw, of from 1000 to 50000 Daltons.

10. A process of making the macromonomer defined in any one of the previous claims comprising the steps of
i) reacting an unsaturated non-mineral oil with an enophile having an acid, ester or anhydride moiety in the presence of iodine to form an adduct
ii) reacting the adduct with an ethylenically unsaturated monomer having a moiety reactive with the acid, ester or anhydride moiety of the enophile to form a low molecular weight monomer
iii) reacting the monomer of step ii) with a chain extender material having at least two moieties reactive with the acid, ester or anhydride of the enophile.

11. An addition polymer derived from an ethylenically unsaturated monomer composition comprising the unsaturated macromonomer of any one of claims 1 to 9.

12. An addition polymer according to claim 11 wherein the polymer is in the form of an aqueous dispersion of polymer microparticles.

13. A dispersion of polymer microparticles according to claim 11 wherein the microparticles have a core polymer composition differing from the shell polymer composition.

14. A dispersion of polymer microparticles according to claim 12 wherein the core consists of the macromonomer.

15. A dispersion of polymer microparticles according to any one of claims 11 to 14 wherein the core:shell ratio, calculated on a weight basis, is from 1:8 to 2:1.

16. A dispersion of polymer microparticles according to any one of claims 11 to 15 wherein the microparticles have a mean average diameter of from 0.05 microns to 2.00 microns.

17. A dispersion of polymer microparticles according to any one of claims 11 to 16 wherein the Tg of the polymer is from -20 to 120°C.

18. A coating composition comprising the aqueous dispersion of any one of claims 12 to 17.

19. A coating composition according to claim 18 wherein the composition further contains ingredients selected from the group comprising pigments, fillers, waxes, extenders, rheological modifiers, dispersants, anti-foams, tackifiers, plasticisers, flow aids and biocides.
